# EUROPEAN PATENT SPECIFICATION

(11) **EP 3 107 440 B1**
(45) Date of publication and mention of the grant of the patent: **06.04.2022**
(21) Application number: 15751809.3
(22) Date of filing: 19.02.2015
(51) Int. Cl.: A61B 1/00, A61B 1/07

(54) **ENDOSCOPE ILLUMINATION SYSTEM AND METHOD FOR SHADOW CREATION AND IMPROVED DEPTH PERCEPTION AND EDGE DETECTION**
ENDOSKOPBELEUCHTUNGSSYSTEM UND VERFAHREN FÜR SCHATTENERZEUGUNG UND VERBESSERTE TIEFENWAHRNEHMUNG UND RANDERKENNUNG
SYSTÈME D'ÉCLAIRAGE POUR ENDOSCOPE ET PROCÉDÉ DE CRÉATION D'OMBRE ET PERCEPTION AMÉLIORÉE DE LA PROFONDEUR ET DE DÉTECTION AMÉLIORÉE DES CONTOURS

(30) Priority: 20.02.2014 US 201461942454 P
(43) Date of publication of application: 28.12.2016
(73) Proprietor: Steris Instrument Management Services, Inc., Birmingham, AL 35222 (US)
(72) Inventor: DYBIEC, Maciej, Cooper City, FL 33328 (US); BODOR, Peter, Pal, Pembroke Pines, FL 33029 (US)
(74) Representative: Range, Christopher William
(86) International application number: PCT/US2015/016632
(87) International publication number: WO 2015/127090

(56) References cited:
- WO-A1-01/56457
- WO-A1-2014/006549
- DE-C1- 19 532 095
- JP-B2- H07 104 493
- US-A1- 2009 203 966
- US-A1- 2010 048 995
- US-A1- 2012 041 267
- US-A1- 2013 023 732
- US-A1- 2013 071 077
- US-A1- 2013 296 652
- None

## Description

### Technical Field

The present invention is directed to an endoscope illumination system and method of using same. More particularly, the present invention is directed to a directional illumination system for use with an endoscope for creating shadows in an object field for improving depth perception and contrast.

### Background of Invention

Artificial shadow creation methods exist in the field of optics. For example, artificial shadow creation is used in metrology to measure depth and in machine vision applications.

US-A-2013/0071077 relates to an endoscope connector method and apparatus. Two illumination sources can generate light and can focus the generated light into two illumination fibres respectively which can guide the light to the distal end of the probe of the working assembly. US2012/041267 relates to an endoscope system for enhanced visualization.

### Summary of the Invention

The present invention is directed to a directional illumination system for use with an endoscope for creating shadows in an object field for improving depth perception and contrast. According to one aspect of the invention, there is provided an endoscope illumination system according to claim 1.

In use, a front end of the endoscope distal tip is faced towards an object to be illuminated and a shadow is created that extends from the object by activating the first set of illumination fibers without activating the second set of illumination fibers. Creation of the shadow improves a user's depth perception within the object field. The endoscope illumination system can also be used to improve edge detection of an area of stained biological tissue by facing a front end of the endoscope distal tip towards the area of stained tissue and activating the first set of illumination fibers without activating the second set of illumination fibers. Thereafter, the second set of illumination fibers is activated without activating the first set of illumination fibers. Depending on the type of dye used to stain the tissue, the at least one light source may emits visual spectrum light or non-visual spectrum light.

According to yet another aspect of the invention, there is provided a method according to claim 8.

By activating the first selectively activatable illumination member without activating the second selectively activatable illumination member, shadows are created in the object field based upon the morphology of the surface being illuminated. Further, when the surface being illuminated includes stained and unstained areas of biological tissue, activating the first selectively activatable illumination member without activating the second selectively activatable illumination member improves detection of an edge of the area of stained biological tissue in the object field. Edge detection is further improved by activating the second selectively activatable illumination member without activating the first selectively activatable illumination member. Improvement in edge detection comes from direct comparison/overlay of two (first and second selectively activatable illumination) obtained images where the only common feature should be the edge.

### Brief Description of the Figures

FIG. 1 is a perspective view of a front end of an endoscope distal tip in accordance with the present invention displaying multiple light ports for controlled directional illumination.
FIG. 2 is a perspective view of a front end of an endoscope distal tip in accordance with the present invention displaying fiber optic bundles located within the multiple light ports.
FIG. 3 is an elevational view of the front end of the endoscope distal tip of FIG. 1 with schematic drawings illustrating independently activated switch mechanisms and power sources for selectively powering directional illumination bundles that are associated with the lights ports.
FIG. 4A is a perspective view of the endoscope distal tip of FIG. 1 illustrating directional illumination and shadow creation within an object field by selectively powering light bundles to create a leftward extending shadow.
FIG. 4B is a perspective view of the endoscope distal tip of FIG. 1 illustrating directional illumination and shadow creation within an object field by selectively powering light bundles to create a rightward extending shadow.
FIG. 5A is a perspective view of the endoscope distal tip of FIG. 1 illustrating directional illumination and shadow creation within an object field by selectively powering light bundles to create a shadow and expose features that are not visible in direct illumination.
FIG. 5B is a perspective view of the endoscope distal tip of FIG. 1 illustrating directional illumination and shadow creation within an object field by selectively powering light bundles to create a extending shadow.
FIG. 6A is a perspective view of the endoscope distal tip of FIG. 1 illustrating directional illumination within an object field by selectively powering light bundles for enhanced edge detection between a stained area and a non-stained area.
FIG. 6B is a perspective view of the endoscope distal tip of FIG. 1 illustrating directional illumination within an object field by selectively powering light bundles for enhanced edge detection between a stained area and a non-stained area.

### Detailed Description

The present invention is directed to a directional illumination system for creating shadows in an object field for improving depth perception and contrast. Directional illumination is based on the ability to control a light source in a manner that illuminates an object from a predetermined direction, e.g., from a first lateral side, a second lateral side, a top side or a bottom side, in order to create well-defined shadows. Doing so enhances a user's depth perception within the object field. According to the present invention, directional illumination is accomplished by providing multiple light ports that contain respective optical fiber bundles that are connected with respective light sources that can be switched on/off independently, arranging the light ports so that light from the optical fiber bundles strikes an object in an object field at different angles and activating all but at least one of the optical fiber bundles. Directional illumination can also be accomplished by providing a whole circular light bundle that is divided into sections that can be switched on/off independently.

In addition to improving depth perception and contrast, the directional illumination system of the present invention can be used to improve zone differentiation in fluorescence imaging techniques. Delivering light to fluorescent dyes from different angles using the present invention can improve accurate border analysis and detection of stained areas vs. unstained areas in the object field.

The primary filed of application of the directional illumination system of the present invention is endoscopy. Controlled shadow creation can be used in endoscopy to enhance depth perception of three dimensional objects in the object field. Improved depth perception is desired in endoscopy since objects observed through an endoscope typically provide poor depth perception since they can only be observed from the front of the object with very little space between the distal end of the endoscope and object. The present invention is applicable in the imaging of complex surface morphologies where direct frontal illumination, as is currently provided in endoscopy field, might not reveal all the necessary detail. Further, the present invention can improve contrast where surfaces in the object field are shiny and/or reflective by adding or enhancing shadows which expose contours that might not be visible in uniform frontal illumination.

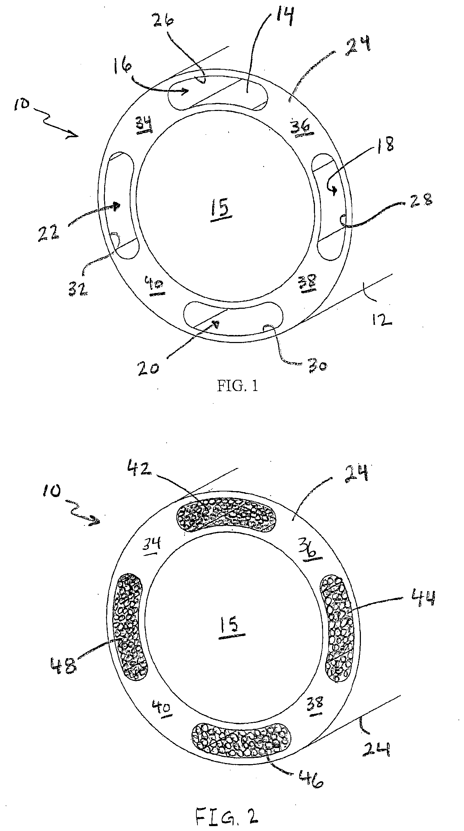

Referring to FIG. 1, there is depicted an endoscope distal tip 10 in accordance with the present invention. Distal tip 10 includes a continuous outer wall 12, a continuous inner wall 14 and a primary channel 15 defined by inner wall 14. Primary channel 15 is typically used to extend surgical instruments through the endoscope and into an object field. Extending between outer wall 12 and inner wall 14 are four illumination channels 16, 18, 20 and 22 that extend longitudinally through distal tip 10 and open through a front face 24 of the distal tip at respective light ports 26, 28, 30 and 32. Illumination channels are sealed from one another by ribs 34, 36, 38 and 40 and arranged equidistantly around front face 24.

Referring to FIG. 2, a plurality of optical fibers extends longitudinally through distal tip 10. The optical fibers are divided into four optical fiber bundles 42, 44, 46 and 48 which extend through respective illumination channels 16, 18, 20 and 22. Optical fiber bundles 42, 44, 46 and 48 are arranged within respective illumination channels 16, 18, 20 and 22 to direct light through respective light ports 26, 28, 30 and 32 and front face 24 of distal tip 10 onto an object field. The placement of light ports 26, 28, 30 and 32 around front face 24 ensures that light emitted by optical fiber bundles 42, 44, 46 and 48 strikes objects with the object field from different angles.

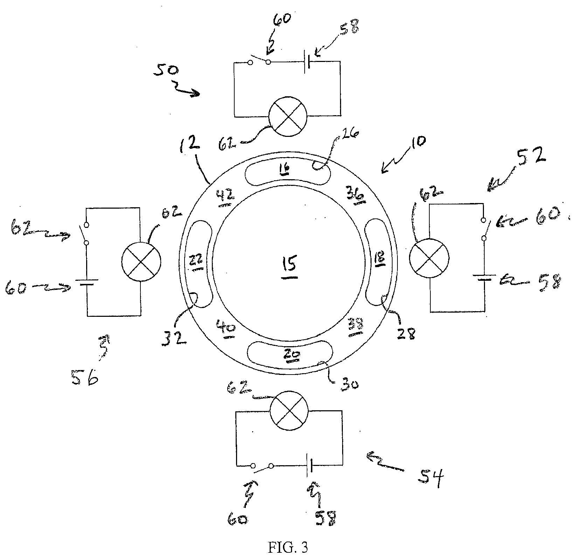

Referring to FIG. 3, optical fiber bundles 42, 44, 46 and 48 receive light from independently controllable light sources 50, 52, 54 and 56 which allows light to be selectively transmitted to and through each of optical fiber bundles 42, 44, 46 and 48. Each of light sources 50, 52, 54 and 56 includes a power source 58, an independently actuatable switch 60 and a light generator 62. Depending on the object field being observed and the surgical procedure being performed, light generator 62 may generate light in the visual spectrum or the non-visual spectrum. In certain instances, it may be beneficial that some of light sources 50, 52, 54 and 56 emit light in the visual spectrum, while other others emit light in the non-visual spectrum. As an alternative to using four independently controllable light sources to provide light independently to each of optical fiber bundles 42, 44, 46 and 48, the present invention may rely upon a single light generator in combination with a light splitter, e.g., one or more prisms, and set of shutters for selectively blocking the transmission of light from the single light generator to the optical fiber bundles.

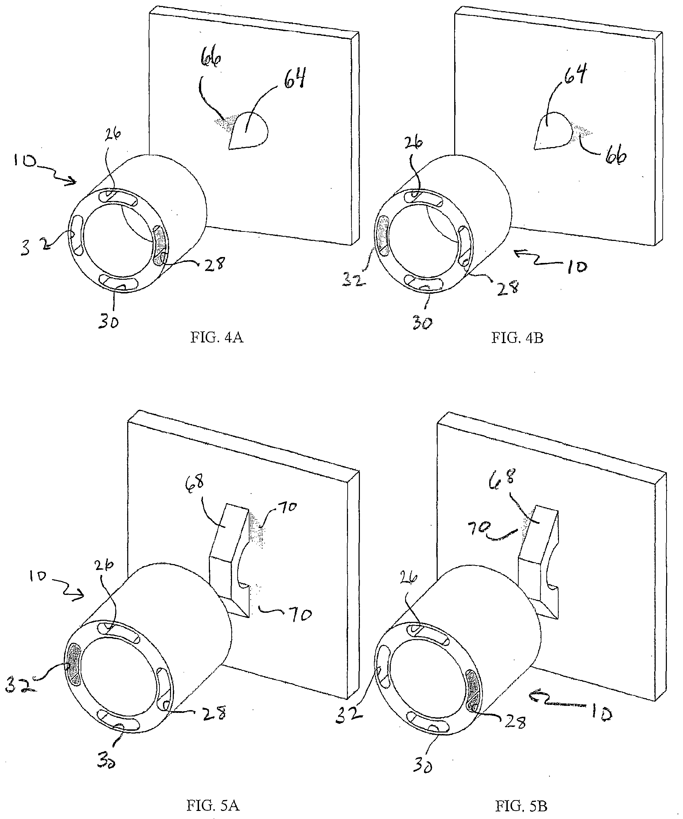

Referring to FIGS. 4A and 4B, there is depicted a method of utilizing a directional illumination system to create shadows in accordance with a first embodiment of the present invention. The method includes providing an endoscope including distal tip 10 with optical fiber bundles 42, 44, 46 and 48 operatively coupled to independently controllable light sources 50, 52, 54 and 56, directing front face 24 of the distal tip toward an object 64 to be illuminated and activating three or less of light generators 62 to thereby emit light from three or less of light ports 26, 28, 30 and 32. As shown in FIG. 4A, light is emitted from a single light port 28, while no light is emitted by light ports, 26, 30 and 32. This accomplished by utilizing switch 60 of light source 52 to activate light generator 62 to transmit light to optical fiber bundle 44, while the switches of light sources 50, 54 and 56 remain in an off position. With light emitting only from light port 28, a shadow 66 is cast that extends leftward from object 64. The existence of shadows 66 adds perspective to the image of object and imparts improved depth perception to the user. Perspective and depth perception are further improved by independently activating other light sources 50, 54 and 56 to form shadows that extend rightward, as depicted in FIG. 4B, upward by activating light source 54 only or downward by activating only light source 50 only. By selectively alternating between the various light sources and thereby the angle by which light strikes object 64, a user can better analyze and visually perceive object 64.

Referring to FIGS. 5A and 5B, there is depicted a method of utilizing a directional illumination system to reveal morphology features that are easily detectable in shadow envelopes in accordance with the present invention. The method includes providing an endoscope including distal tip 10 with optical fiber bundles 42, 44, 46 and 48 operatively coupled to independently controllable light sources 50, 52, 54 and 56, directing front face 24 of the distal tip toward an object 64 to be illuminated and activating three or less of light generators 62 to thereby emit light from three or less of light ports 26, 28, 30 and 32. As shown in FIG. 5A, light is emitted from a single light port 32, while no light is emitted by light port 26, 28 and 30. This accomplished by utilizing switch 60 of light source 56 to activate light generator 62 to transmit light to optical fiber bundle 48, while the switches of light sources 50, 52 and 54 remain in an off position. With light emitting only from light port 32, a shadow 66 is cast that extends rightward from object 64. The existence of shadows 66 adds perspective to the image of object and imparts improved depth perception to the user. Perspective and depth perception are further improved by independently activating other light sources 50, 52 and 54 to form shadows that extend leftward, as depicted in FIG. 5B, upward by activating light source 54 only or downward by activating only light source 50 only. By selectively alternating between the various light sources and thereby the angle by which light strikes object 64, a user can better analyze and visually perceive object 64.

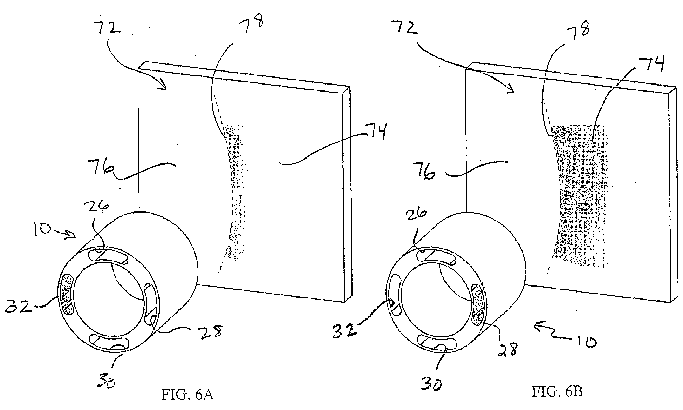

Referring to FIGS. 6A and 6B, there is depicted a method of utilizing a directional illumination system to improve edge detection of a stained biological tissue within an object field in accordance with the present invention. The method includes providing an endoscope including distal tip 10 with optical fiber bundles 42, 44, 46 and 48 operatively coupled to independently controllable light sources 50, 52, 54 and 56, directing front face 24 of the distal tip toward a specimen including a biological tissue that includes a stained portion 74 and an unstained portion 76 and activating three or less of light generators 62 to thereby propagate light from three or less of light ports 26, 28, 30 and 32. As shown in FIG. 6A, light is propagated from a single light port 32, while no light is delivered by light ports, 26, 38 and 30. With light propagating from light port 32, an edge 78 can be observed between the stained portion 74, which emits light due to fluorescence, and the unstained portion 76, which emits little to no light. Additionally, depending on the surface morphology, shadows may be cast adding to the user's depth perception in the object field. Detection and analysis of the of edge 78 is improved by independently activating light source 52, which directs light at edge 78 from different angle than the light emitted from light source 66. With the use of independent light ports 26, 28, 30 and 32, it is possible to create and/or force shadows in one predefined direction that allows for better surface morphology assessment. If a shadow in an expected predefined direction cannot be found, sequential scan with all light ports and consecutive image analysis can be used to determine in what direction surface features are oriented. Analysis of all shadow patterns from sequential illumination provides additional information about feature orientation with respect to the tip of endoscope.

As will be apparent to one skilled in the art, various modifications can be made within the scope of the aforesaid description. Such modifications being within the ability of one skilled in the art form a part of the present invention and are embraced by the claims below.

## Claims

1. An endoscope illumination system comprising:
an endoscope distal tip (10) including a first light port (28), a second light port (32), a third light port (26) and a fourth light port (30), the first light port (28) being arranged on a first lateral side of the endoscope distal tip (10), the second light port (32) being arranged on a second lateral side of the endoscope distal tip (10), the third light port (26) being arranged on a top side of the endoscope distal tip (10) and the fourth light port (30) being arranged on a bottom side of the endoscope distal tip (10),
a first illumination member (44) within the endoscope distal tip (10) that is arranged to direct light through the first light port (28) onto an object field,
a second illumination member (48) within the endoscope distal tip (10) that is arranged to direct light through the second light port (32) onto the object field,
a third illumination member (42) within the endoscope distal tip (10) that is arranged to direct light through the third light port (26) on to the object field,
a fourth illumination member (46) within the endoscope distal tip (10) that is arranged to direct light through the fourth light port (30) onto the object field,
at least one light source operatively coupled to the first illumination member (44) , the second illumination member (48), the third illumination member (42) and the fourth illumination member (46), and
a switching mechanism operatively coupled to the first illumination member (44) , the second illumination member (48), the third illumination member (42), the fourth illumination member (46) and the at least one light source,
wherein the switching mechanism includes a first switch adapted and arranged to selectively activate and deactivate the first illumination member (44) , a second switch adapted and arranged to selectively activate and deactivate the second illumination member (48) independently of activation of the first illumination member (44) by the first switch, a third switch adapted and arranged to selectively activate and deactivate the third illumination member (42) independently of activation of the first illumination member (44) by the first switch, and a fourth switch adapted and arranged to selectively activate and deactivate the fourth illumination member (46) independently of activation of the first illumination member (44) by the first switch;
wherein the distal tip 10 further comprises a continuous outer wall (12), a continuous inner wall (14) and a primary channel (15) defined by the inner wall (14); and extending longitudinally through the distal tip between the outer wall and the inner wall, four illumination channels that open through a front face of the distal tip at the respective first to fourth light ports, **characterized in that** the continuous outer wall (12), the continuous inner wall (14) and the primary channel (15) are arranged coaxially.

2. The endoscope illumination system according to claim 1 wherein the at least one light source includes a first light source operatively coupled to the first illumination member (44) , a second light source operatively coupled to the second illumination member (48), a third light source operatively coupled to the third illumination member (42) and a fourth light source operatively coupled to the fourth illumination member (46).

3. The endoscope illumination system according to claim 1 wherein the switching mechanism is configured for independently activating each of the first light, the second light source, the third light source and the fourth light source.

4. The endoscope illumination system according to any preceding claim wherein the at least one light source includes a visual spectrum light source, a non-visual spectrum light source or both the visual spectrum light source and the non-visual spectrum light source.

5. The endoscope illumination system according to claim 1 wherein the first illumination member (44) includes optical fibers.

6. The endoscope illumination system according to claim 1 wherein the illumination channels (16, 18, 20, 22) are arranged equidistantly around a front face (24) of the distal tip (10).

7. The endoscope illumination system according to any preceding claim wherein the second illumination member (48) includes optical fibers.

8. A method of illuminating an object for non-surgical purposes comprising providing an endoscope including the endoscope illumination system of any of claims 1 to 7, facing a front end of the endoscope distal tip (10) towards the object and creating a shadow extending from the object by activating the first illumination member (44) without activating the second, third or fourth illumination member (46), then activating the second illumination member (48) without activating the first, third or fourth illumination member (46), then activating the third illumination member (42) without activating the first, second or fourth illumination member (46), then activating the fourth illumination member (46) without activating the first, second or third illumination member (42).

## Patentansprüche

1. Endoskopbeleuchtungssystem, umfassend:
eine distale Endoskopspitze (10) mit einer ersten Lichtöffnung (28), einer zweiten Lichtöffnung (32), einer dritten Lichtöffnung (26) und einer vierten Lichtöffnung (30), wobei die erste Lichtöffnung (28) an einer ersten lateralen Seite der distalen Endoskopspitze (10) angeordnet ist, die zweite Lichtöffnung (32) an einer zweiten lateralen Seite der distalen Endoskopspitze (10) angeordnet ist, die dritte Lichtöffnung (26) an einer Oberseite der distalen Endoskopspitze (10) angeordnet ist und die vierte Lichtöffnung (30) an einer Unterseite der distalen Endoskopspitze (10) angeordnet ist,
ein erstes Beleuchtungselement (44) innerhalb der distalen Endoskopspitze (10), das angeordnet ist, um Licht durch die erste Lichtöffnung (28) auf ein Objektfeld zu richten,
ein zweites Beleuchtungselement (48) innerhalb der distalen Endoskopspitze (10), das angeordnet ist, um Licht durch die zweite Lichtöffnung (32) auf das Objektfeld zu richten,
ein drittes Beleuchtungselement (42) innerhalb der distalen Endoskopspitze (10), das angeordnet ist, um Licht durch die dritte Lichtöffnung (26) auf das Objektfeld zu richten,
ein viertes Beleuchtungselement (46) innerhalb der distalen Endoskopspitze (10), das angeordnet ist, um Licht durch die vierte Lichtöffnung (30) auf das Objektfeld zu richten,
mindestens eine Lichtquelle, die mit dem ersten Beleuchtungselement (44), dem zweiten Beleuchtungselement (48), dem dritten Beleuchtungselement (42) und dem vierten Beleuchtungselement (46) wirkverbunden ist, und
einen Schaltmechanismus, der mit dem ersten Beleuchtungselement (44), dem zweiten Beleuchtungselement (48), dem dritten Beleuchtungselement (42), dem vierten Beleuchtungselement (46) und der mindestens einen Lichtquelle wirkverbunden ist,
wobei der Schaltmechanismus einen ersten Schalter umfasst, der ausgelegt und eingerichtet ist, um das erste Beleuchtungselement (44) selektiv zu aktivieren und zu deaktivieren, einen zweiten Schalter, der ausgelegt und eingerichtet ist, um das zweite Beleuchtungselement (48) unabhängig von der Aktivierung des ersten Beleuchtungselements (44) durch den ersten Schalter selektiv zu aktivieren und zu deaktivieren, einen dritten Schalter, der ausgelegt und eingerichtet ist, um das dritte Beleuchtungselement (42) unabhängig von der Aktivierung des ersten Beleuchtungselements (44) durch den ersten Schalter selektiv zu aktivieren und zu deaktivieren, und einen vierten Schalter, der ausgelegt und eingerichtet ist, um das vierte Beleuchtungselement (46) unabhängig von der Aktivierung des ersten Beleuchtungselements (44) durch den ersten Schalter selektiv zu aktivieren und zu deaktivieren;
wobei die distale Spitze (10) des Weiteren eine durchgehende Außenwand (12), eine durchgehende Innenwand (14) und einen durch die Innenwand (14) definierten primären Kanal (15) aufweist; und in Längsrichtung durch die distale Spitze zwischen der Außenwand und der Innenwand vier Beleuchtungskanäle verlaufen, die sich durch eine Vorderfläche der distalen Spitze hindurch an den jeweiligen ersten bis vierten Lichtöffnungen öffnen, **dadurch gekennzeichnet, dass** die durchgehende Außenwand (12), die durchgehende Innenwand (14) und der primäre Kanal (15) koaxial angeordnet sind.

2. Endoskopbeleuchtungssystem nach Anspruch 1, bei dem die mindestens eine Lichtquelle eine erste Lichtquelle, die mit dem ersten Beleuchtungselement (44) wirkverbunden ist, eine zweite Lichtquelle, die mit dem zweiten Beleuchtungselement (48) wirkverbunden ist, eine dritte Lichtquelle, die mit dem dritten Beleuchtungselement (42) wirkverbunden ist, und eine vierte Lichtquelle, die mit dem vierten Beleuchtungselement (46) wirkverbunden ist, umfasst.

3. Endoskopbeleuchtungssystem nach Anspruch 1, bei dem der Schaltmechanismus konfiguriert ist, um die erste Lichtquelle, die zweite Lichtquelle, die dritte Lichtquelle und die vierte Lichtquelle jeweils unabhängig voneinander zu aktivieren.

4. Endoskopbeleuchtungssystem nach einem der vorhergehenden Ansprüche, bei dem die mindestens eine Lichtquelle eine Lichtquelle im visuellem Spektrum, eine Lichtquelle im nicht-visuellem Spektrum, oder sowohl die Lichtquelle im visuellem Spektrum als auch die Lichtquelle im nicht-visuellem Spektrum umfasst.

5. Endoskopbeleuchtungssystem nach Anspruch 1, bei dem das erste Beleuchtungselement (44) Lichtleitfasern enthält.

6. Endoskopbeleuchtungssystem nach Anspruch 1, bei dem die Beleuchtungskanäle (16, 18, 20, 22) gleichmäßig beabstandet um eine Stirnfläche (24) der distalen Spitze (10) herum angeordnet sind.

7. Endoskopbeleuchtungssystem nach einem der vorhergehenden Ansprüche, bei dem das zweite Beleuchtungselement (48) Lichtleitfasern enthält.

8. Verfahren zur Beleuchtung eines Objekts für nicht-chirurgische Zwecke, umfassend das Bereitstellen eines Endoskops mit dem Endoskopbeleuchtungssystem nach einem der Ansprüche 1 bis 7, das Ausrichten eines vorderen Endes der distalen Endoskopspitze (10) auf das Objekt und das Erzeugen eines von dem Objekt weg verlaufenden Schattens durch Aktivierung des ersten Beleuchtungselements (44) ohne Aktivierung des zweiten, dritten oder vierten Beleuchtungselements (46), anschließende Aktivierung des zweiten Beleuchtungselements (48) ohne Aktivierung des ersten, dritten oder vierten Beleuchtungselements (46), anschließende Aktivierung des dritten Beleuchtungselements (42) ohne Aktivierung des ersten, zweiten oder vierten Beleuchtungselements (46), anschließende Aktivierung des vierten Beleuchtungselements (46) ohne Aktivierung des ersten, zweiten oder dritten Beleuchtungselements (42).

## Revendications

1. Système d'éclairage d'endoscope comprenant:
une pointe distale d'endoscope (10) incluant un premier orifice de lumière (28), un second orifice de lumière (32), un troisième orifice de lumière (26) et un quatrième orifice de lumière (30), le premier orifice de lumière (28) étant agencé sur un premier côté latéral de la pointe distale d'endoscope (10), le second orifice de lumière (32) étant agencé sur un second côté latéral de la pointe distale d'endoscope (10), le troisième orifice de lumière (26) étant agencé sur un côté supérieur de la pointe distale d'endoscope (10) et le quatrième orifice de lumière (30) étant agencé sur un côté inférieur de la pointe distale d'endoscope (10),
un premier élément d'éclairage (44) à l'intérieur de la pointe distale d'endoscope (10) qui est agencé pour diriger la lumière à travers le premier orifice de lumière (28) sur un champ objet,
un second élément d'éclairage (48) à l'intérieur de la pointe distale d'endoscope (10) qui est agencé pour diriger la lumière à travers le second orifice de lumière (32) sur le champ objet,
un troisième élément d'éclairage (42) à l'intérieur de la pointe distale d'endoscope (10) qui est agencé pour diriger la lumière à travers le troisième orifice de lumière (26) sur le champ objet,
un quatrième élément d'éclairage (46) à l'intérieur de la pointe distale d'endoscope (10) qui est agencé pour diriger la lumière à travers le quatrième orifice de lumière (30) sur le champ objet,
au moins une source de lumière couplée de manière fonctionnelle au premier élément d'éclairage (44), au second élément d'éclairage (48), au troisième élément d'éclairage (42) et au quatrième élément d'éclairage (46), et
un mécanisme de commutation couplé de manière fonctionnelle au premier élément d'éclairage (44), au second élément d'éclairage (48), au troisième élément d'éclairage (42), au quatrième élément d'éclairage (46) et à la au moins une source de lumière,
dans lequel le mécanisme de commutation inclut un premier commutateur adapté et agencé pour activer et désactiver sélectivement le premier élément d'éclairage (44), un second commutateur adapté et agencé pour activer et désactiver sélectivement le second élément d'éclairage (48) indépendamment de l'activation du premier élément d'éclairage (44) par le premier commutateur, un troisième commutateur adapté et agencé pour activer et désactiver sélectivement le troisième élément d'éclairage (42) indépendamment de l'activation du premier élément d'éclairage (44) par le premier commutateur, et un quatrième commutateur adapté et agencé pour activer et désactiver sélectivement le quatrième élément d'éclairage (46) indépendamment de l'activation du premier élément d'éclairage (44) par le premier commutateur;
dans lequel la pointe distale 10 comprend en outre une paroi externe continue (12), une paroi interne continue (14) et un canal primaire (15) défini par la paroi interne (14); et s'étendant longitudinalement à travers la pointe distale entre la paroi externe et la paroi interne, quatre canaux d'éclairage qui ouvrent à travers une face avant de la pointe distale au niveau des premier à quatrième orifices de lumière respectifs, **caractérisé en ce que** la paroi externe continue (12), la paroi interne continue (14) et le canal primaire (15) sont agencés coaxialement.

2. Système d'éclairage d'endoscope selon la revendication 1, dans lequel la au moins une source de lumière inclut une première source de lumière couplée de manière fonctionnelle au premier élément d'éclairage (44), une seconde source de lumière couplée de manière fonctionnelle au second élément d'éclairage (48), une troisième source de lumière couplée de manière fonctionnelle au troisième élément d'éclairage (42) et une quatrième source de lumière couplée de manière fonctionnelle au quatrième élément d'éclairage (46).

3. Système d'éclairage d'endoscope selon la revendication 1, dans lequel le mécanisme de commutation est configuré pour activer indépendamment chacune de la première lumière, de la seconde source de lumière, de la troisième source de lumière et de la quatrième source de lumière.

4. Système d'éclairage d'endoscope selon l'une quelconque des revendications précédentes, dans lequel la au moins une source de lumière inclut une source de lumière du spectre visuel, une source de lumière du spectre non visuel ou à la fois la source de lumière du spectre visuel et la source de lumière du spectre non visuel.

5. Système d'éclairage d'endoscope selon la revendication 1, dans lequel le premier élément d'éclairage (44) inclut des fibres optiques.

6. Système d'éclairage d'endoscope selon la revendication 1, dans lequel les canaux d'éclairage (16, 18, 20, 22) sont agencés à égale distance autour d'une face avant (24) de la pointe distale (10).

7. Système d'éclairage d'endoscope selon l'une quelconque des revendications précédentes, dans lequel le second élément d'éclairage (48) inclut des fibres optiques.

8. Procédé d'éclairage d'un objet à des fins non chirurgicales comprenant la fourniture d'un endoscope incluant le système d'éclairage d'endoscope selon l'une quelconque des revendications 1 à 7, le fait d'orienter une extrémité avant de la pointe distale d'endoscope (10) vers l'objet et la création d'une ombre s'étendant depuis l'objet par activation du premier élément d'éclairage (44) sans activation du second, troisième ou quatrième élément d'éclairage (46), puis l'activation du second élément d'éclairage (48) sans activation du premier, troisième ou quatrième élément d'éclairage (46), puis l'activation du troisième élément d'éclairage (42) sans activation du premier, second ou quatrième élément d'éclairage (46), puis l'activation du quatrième élément d'éclairage (46) sans activation du premier, second ou troisième élément d'éclairage (42).
